# NEUE EUROPÄISCHE PATENTSCHRIFT

(11) **EP 2 877 441 B2**
(45) Veröffentlichungstag und Bekanntmachung des Hinweises auf die Entscheidung über den Einspruch: **16.06.2021**
(45) Hinweis auf die Patenterteilung: 14.09.2016
(21) Anmeldenummer: 13739715.4
(22) Anmeldetag: 23.07.2013
(51) Int. Cl.: C07C 201/08

(54) **VERFAHREN ZUR HERSTELLUNG VON NITROBENZOL DURCH ADIABATE NITRIERUNG**
METHOD FOR MANUFACTURING NITROBENZOL THROUGH ADIABATIC NITRATION
PROCÉDÉ DE FABRICATION DE NITROBENZOLE PAR NITRIFICATION ADIABATE

(30) Priorität: 27.07.2012 EP 12178159
(43) Veröffentlichungstag der Anmeldung: 03.06.2015
(73) Patentinhaber: Covestro Intellectual Property GmbH & Co. KG, 51373 Leverkusen (DE)
(72) Erfinder: KNAUF, Thomas, 41542 Dormagen (DE); MERKEL, Michael, 40223 Düsseldorf (DE); MAIRATA, Antoni, 40549 Düsseldorf (DE)
(74) Vertreter: Levpat
(86) Internationale Anmeldenummer: PCT/EP2013/065504
(87) Internationale Veröffentlichungsnummer: WO 2014/016290

(56) Entgegenhaltungen:
- EP-A1- 2 070 907
- EP-B1- 0 976 718

## Beschreibung

Gegenstand der Erfindung ist ein kontinuierliches adiabates Verfahren zur Herstellung von Nitrobenzol durch Nitrierung von Benzol mit Mischungen aus Schwefel- und Salpetersäure unter Einsatz eines stöchiometrischen Überschusses an Benzol, bei dem der Gehalt an organischen Verbindungen in der Kreislaufschwefelsäure mindestens während der Anfahrzeit der Produktionsanlage stets kleiner als 1,0 Massen-%, bezogen auf die Gesamtmasse der Kreislaufschwefelsäure, gehalten wird. Dies wird erreicht, indem entweder nach Beendigung oder vor Beginn eines Produktionszyklus die Kreislaufschwefelsäure bei erhöhter Temperatur im Kreis gefahren wird, und so im Verdampfungsapparat für die Aufkonzentrierung der Schwefelsäure die in der Schwefelsäure enthaltenen Organika, bevorzugt umfassend Nitrobenzol und Spuren an Benzol, Dinitrobenzol und Nitrophenole, abgetrennt werden.

Die vorliegende Erfindung betrifft ein kontinuierliches Verfahren zur Herstellung von Nitrobenzol durch adiabate Nitrierung von Benzol durch ein Gemisch von Schwefel- und Salpetersäure (sog. Mischsäure). Ein solches Verfahren wurde erstmals in US 2,256,999 beansprucht und ist in moderneren Ausführungsformen in US 4,091,042, US 5,313,009 und US 5,763,697 beschrieben.

Den beschriebenen adiabaten Verfahren ist gemein, dass die Ausgangsstoffe Benzol und Salpetersäure in einem großen Überschuss an Schwefelsäure zur Reaktion gebracht werden, welche die frei werdende Reaktionswärme und das bei der Reaktion gebildete Wasser aufnimmt.

Die Reaktionsführung erfolgt im Allgemeinen so, dass die Salpetersäure und Schwefelsäure zur sogenannten Nitriersäure (auch Mischsäure genannt) vereint werden. Benzol wird in diese Nitriersäure hinein dosiert. Die Reaktionsprodukte sind im Wesentlichen Wasser und Nitrobenzol. In der Nitrierreaktion wird Benzol bezogen auf die molare Salpetersäuremenge mindestens in stöchiometrischer Menge, aber bevorzugt in 2%igem bis 10%igem Überschuss eingesetzt. Das in den Reaktionsapparaten gebildete und im Phasentrennapparat von der Säurephase abgetrennte Rohnitrobenzol wird gemäß dem Stand der Technik einer Wäsche und einer destillativen Aufarbeitung unterzogen, wie beispielsweise in EP 1 816 117 A1 (Seite 2, Zeile 26 bis 42), US 4,091,042 (siehe oben) oder US 5,763,697 (siehe oben) beschrieben. Charakteristisch für diese Aufarbeitung ist, dass nicht umgesetztes überschüssiges Benzol nach der Wäsche von Nitrobenzol in einer abschließenden Destillation abgetrennt wird und als Rückbenzol, das auch niedrig siedende, nichtaromatische organische Verbindungen (sog. Leichtsieder) enthält (siehe DE 10 2009 005 324 A1), wieder in der Nitrierreaktion eingesetzt wird. Die Behandlung des Abgases aus der adiabaten Nitrierreaktion wird in EP 0 976 718 B1 beschrieben. Das Abgas aus Säurekreislauf und fertigem Rohnitrobenzol wird abgezogen, vereint und über einen NOx-Absorber geschickt, um verdünnte Salpetersäure zurückzugewinnen, die in die Reaktion zurückgefahren wird. Die als Kreislaufsäure bezeichnete Schwefelsäure wird in einem Blitzverdampfer aufkonzentriert und weitestgehend von Organika befreit. Es verbleiben hochsiedende Organika wie z. B. Nitrobenzol, Dinitrobenzol und Nitrophenole und in Spuren Benzol in der Kreislaufsäure und werden somit auch zur Reaktion zurückgefahren.

Wenn das Abgas aus einer adiabaten Nitrierreaktion, wie in EP 0 976 718 B1 beschrieben, aufgearbeitet wird, nämlich das Abgas aus Säurekreislauf und fertigem Rohnitrobenzol abgezogen, vereint und durch einen NOx-Absorber geleitet wird, um verdünnte Salpetersäure zurückzugewinnen, die in die Reaktion zurückgefahren wird, dann empfiehlt es sich, diese verdünnte Salpetersäure erst dann wieder in die Reaktion zurückzuführen, wenn der Anfahrvorgang (damit ist der Zeitraum, innerhalb dessen eine Produktionsanlage ausgehend von einem Stillstand auf Soll-Last gefahren wird, gemeint; für Details siehe weiter unten) beendet ist, da sich durch die Beimischung der verdünnten Salpetersäure die Gesamtkonzentration der Einsatzsalpetersäure abschwächt, was zu einer Verlangsamung der Reaktionskinetik führt.

Die Güte eines adiabaten Verfahrens zur Nitrierung aromatischer Kohlenwasserstoffe ist einerseits definiert durch den Gehalt des Produktes an unerwünschten Nebenprodukten der Reaktion, die durch mehrfache Nitrierung oder Oxidation des aromatischen Kohlenwasserstoffes oder des Nitroaromaten gebildet werden. Das Bestreben bei der Herstellung von Nitrobenzol ist es, den Gehalt an Dinitrobenzol und an Nitrophenolen, insbesondere des als brisant einzustufenden Trinitrophenols (Pikrinsäure), zu minimieren. Andererseits ist die Güte eines adiabaten Verfahrens dadurch definiert, dass der Prozess ohne technischen Produktionsausfall betrieben werden kann.

Um Nitrobenzol mit besonders hohen Selektivitäten zu erhalten, wurde die Art der zu verwendenden Mischsäure detailliert festgelegt (EP 0 373 966 B1, EP 0 436 443 B1 und EP 0 771 783 B1), sowie darauf hingewiesen, dass der Gehalt an Nebenprodukten durch die Höhe der Maximaltemperatur bestimmt wird (EP 0 436 443 B1, Spalte 15, Z. 22 bis 25). Auch ist bekannt, dass ein hoher Anfangsumsatz vorteilhaft ist für eine hohe Selektivität und dass diese dann erreicht wird, wenn zu Beginn der Reaktion eine optimale Durchmischung herbeigeführt wird (EP 0 771 783 B1, Absatz [0014]).

Zu hervorragenden Selektivitäten gelangt man, wenn die Reaktionsstarttemperatur sehr niedrig gewählt wird (WO2010/051616 A1), was aber gleichbedeutend mit einer mehrfach verlängerten Reaktionszeit ist. Eine hohe Raum-Zeit-Ausbeute ist für die industrielle Anwendung eines Verfahrens von Vorteil, da dadurch kompakte Reaktionseinrichtungen gebaut werden können, die sich durch ein geringes Investitionsvolumen pro Kapazität auszeichnen. Daher ist diese Vorgehensweise kontraproduktiv.

Allen aufgeführten Literaturstellen gemein ist, dass sie den Anfahrprozess einer Nitrieranlage und seine Schwierigkeiten nicht beschreiben.

Bezüglich der Qualität des Hilfsstoffes Schwefelsäure auf die adiabate Herstellung von Nitrobenzol beschreibt EP 2 070 907 A1, dass sich niedrige Gehalte an Metallionen in der aus der Nitrierung erhaltenen Schwefelsäure positiv auf die Aufkonzentrierung der Schwefelsäure auswirken. So werden bei der Blitzverdampfung (auch Flash-Verdampfung genannt, d. h., eine mit einer Entspannung verbundene Verdampfung) der schwefelsäurehaltigen Absäure, die nach Abtrennung der wässrigen Phase aus dem aus der Nitrierung von Benzol erhaltenen Reaktionsgemisch erhalten wird, höhere Schwefelsäurekonzentrationen in der gewonnenen aufkonzentrierten Schwefelsäure erreicht, wenn der Gehalt an Metallionen niedrig ist. Dies ist vermutlich auf die verbesserte Verdampfbarkeit des Wassers im Blitzverdampfer bei niedrigen Gehalten an Metallionen in der Absäure zurückzuführen. So wurde gefunden, dass bei der Blitzverdampfung unter ansonsten identischen Bedingungen (gleiche Temperatur der Absäure, gleicher Schwefelsäure-Gehalt der Absäure, gleicher Druck im Blitzverdampfer) eine konzentrierte Schwefelsäure mit einer um bis zu 0,25 % höheren Konzentration an H₂SO₄ erhalten wird, wenn eine Absäure mit niedrigen Gehalten an Metallionen von unter 900 mg/l eingesetzt wird.

Weiter beschreibt EP 2 070 907 A1, dass niedrigere Metallionenkonzentrationen in der Schwefelsäure auch zu einer Siedetemperaturerniedrigung der Schwefelsäure führen, wodurch sich ebenfalls ein niedrigerer Bedarf an Energie zur Aufkonzentrierung der Schwefelsäure ergibt.

In EP 2 070 907 A1 wird auch beobachtet, dass die problematischen Ablagerungen von Metallsulfaten nicht nur in Wärmeaustauschern auftreten können, sondern auch an allen Stellen, an denen die Konzentration der schwerlösliche Metallsulfate bildenden Metallionen hoch genug und die Temperatur niedrig genug ist, um zu einer Feststoffbildung zu führen und gleichzeitig die Fließgeschwindigkeit der Schwefelsäure oder der Querschnitt der Schwefelsäure führenden Leitungen niedrig genug ist, um eine für das Verfahren störende Ansammlung der Metallsulfate herbeizuführen. Daher können Ablagerungen von Metallsulfaten nicht nur in Wärmetauschern beobachtet werden, sondern auch als Ablagerungen auf den Böden von Tanks, an Messstellen wie Standmessungen und an Dispergierelementen, die bestimmungsgemäß kleine Durchlassöffnungen aufweisen, auftreten. Ebenso können Ablagerungen von Metallsulfaten auch innerhalb der Blitzverdampfer auftreten, in denen bestimmungsgemäß die Schwefelsäure abgekühlt wird, während Wasser verdampft und die Konzentration der Säure erhöht wird. Des Weiteren können Ablagerungen von Metallsalzen auch in den der Reaktion nachfolgenden Aufarbeitungsschritten, wie zum Beispiel in der Abwasseraufarbeitung, durch mitgeschleppte Metallsulfate entstehen. Um den störenden Einfluss dieser Ablagerungen zu verringern, ist gemäß dem oben zitierten Stand der Technik eine periodische Reinigung der betroffenen Anlagenteile vorgesehen und notwendig. Diese Reinigung ist jedoch mit Stillständen in der Produktion und damit mit zusätzlichen Kosten verbunden. Es kann auf eine Reinigung von Wärmeaustauschern und Schwefelsäure führenden Leitungen von ausgefallenen festen Metallsulfaten verzichtet werden, wenn bei der Nitrierung des Benzols durch eine schwefel- und salpetersäurehaltige Mischsäure die durch Blitzverdampfung von Wasser wiedererhaltene Schwefelsäure nicht vollständig als Kreislaufsäure in die Reaktionszone zurückgeführt wird, sondern teilweise ausgeschleust und durch frische, metallionenarme Schwefelsäure ersetzt wird.

In EP 2 070 907 A1 wird nicht auf organische Verbindungen, die sich in der Kreislaufschwefelsäure, vor allen Dingen wenn die Produktionsanlage mit hohen Lasten betrieben wird, aufkonzentrieren können, eingegangen.

Den beschriebenen Verfahren des Standes der Technik gelingt es zwar, ein Nitrobenzol herzustellen, das einen geringen Gehalt an Nebenprodukten aufweist, also nur von etwa 100 ppm bis 300 ppm an Dinitrobenzol und 1500 ppm bis 2500 ppm an Nitrophenolen enthält, wobei Pikrinsäure einen Anteil von 10 Massen-% bis 50 Massen-% der Nitrophenole annehmen kann. Die Verfahren zeichnen sich auch durch eine hohe Raum-/Zeitausbeute aus. Es werden jedoch nur Verfahren beschrieben, die schon in Gang sind, d. h., bei denen der Zeitraum von Beginn der Reaktion bis zum Erreichen der Soll-Last (sog. "Anfahrzeit") bereits verstrichen ist. Evtl. besondere Schwierigkeiten beim Anfahren eines adiabaten Nitrierprozesses werden nicht angesprochen.

Ausgangspunkt für die vorliegende Erfindung war die Erkenntnis, dass Verunreinigungen in den Ausgangsstoffen oder Hilfsstoffen, insbesondere Verunreinigungen in der verwendeten Kreislaufschwefelsäure, sich während der Anfahrzeit in besonderem Maße negativ auf das Verfahren auswirken.

Organische Verunreinigungen wie Nitrobenzol, Dinitrobenzol und Nitrophenole in der Kreislaufschwefelsäure erniedrigen die insgesamt verfügbare Konzentration an Salpetersäure. Hierdurch wird die Reaktion zwar nicht verlangsamt, aber die verringerte Salpetersäurekonzentration kann zur Folge haben, dass eine zu große Menge an Benzol eingesetzt wird. Dies erhöht den Energieverbrauch bei der Aufarbeitung des Rohnitrobenzols, weil dieses überschüssige Benzol nach der Nitrierung abgekühlt werden muss und bei der destillativen Reinigung von Nitrobenzol abgetrennt werden muss. Außerdem verringern sich durch das zusätzliche Benzol die Verweilzeiten in allen Apparaten, was zu einer Verlangsamung der Startreaktion führt. Außerdem kommt es zu einer Erhöhung von Nebenprodukten, weil organische Verunreinigungen wie Nitrobenzol mit Salpetersäure weiter aufnitriert werden.

Dem vorstehend Gesagten Rechnung tragend ist ein Gegenstand der vorliegenden Erfindung ein Verfahren zur Herstellung von Nitrobenzol, bei dem besonderes Augenmerk auf den kritischen Zeitraum des Anfahrens der Reaktion gelegt wird. Insbesondere wurde gefunden, dass durch eine Beschränkung des Gehaltes an organischen Verbindungen in der Kreislaufschwefelsäure mindestens während der Anfahrzeit die zuvor genannten Schwierigkeiten überwunden oder zumindest deutlich vermindert werden. Diese Beschränkung des Gehaltes an organischen Verbindungen in der Kreislaufschwefelsäure mindestens während der Anfahrzeit ist auf verschiedene Weisen realisierbar, die Gegenstände der vorliegenden Erfindung sind.

Insbesondere ist ein Gegenstand der vorliegenden Erfindung ein kontinuierliches Verfahren zur Herstellung von Nitrobenzol durch Nitrierung von Benzol, bei dem
a) ein Benzol-haltiger Strom (a.1), der bevorzugt mindestens 90 Massen-%, besonders bevorzugt mindestens 95 Massen-%, und ganz besonders bevorzugt mindestens 99 Massen-% Benzol, jeweils bezogen auf die Gesamtmasse von (a.1), umfasst, in einem Reaktor mit einem Gemisch aus Schwefelsäure (a.2) und Salpetersäure (a.3) unter adiabaten Bedingungen umgesetzt wird, wobei Benzol bevorzugt in einem stöchiometrischen Überschuss bezogen auf Salpetersäure (a.3) von 2,0 % bis 20 %, besonders bevorzugt von 5,0 % bis 10 % der Theorie eingesetzt wird, und wobei die Menge M' des dem Reaktor pro Stunde zugeführten Benzol-haltigen Stroms (a.1) innerhalb eines Zeitraums t von Beginn der Nitrierung bis zum Erreichen eines vorgegebenen Soll-Werts für M' gesteigert wird,
b) das in Schritt a) erhaltene Verfahrensprodukt in einem Phasentrennapparat in eine wässrige, Schwefelsäure umfassende Phase (b.1) und eine organische, Nitrobenzol umfassende Phase (b.2) getrennt wird,
c) die in Schritt b) erhaltene wässrige Phase (b.1) durch Verdampfung von Wasser in einem Verdampfungsapparat (dem sog. "Blitzverdampfer") zu einer wässrigen Schwefelsäurehaltigen Phase (c.1) mit gegenüber (b.1) erhöhter Schwefelsäurekonzentration aufkonzentriert wird, und wobei die Phase (c.1) über einen Schwefelsäuretank in Schritt a) zurückgeführt und als Bestandteil von (a.2) eingesetzt wird,
d) die in Schritt b) erhaltene organische Phase (b.2) zu Rein-Nitrobenzol (d.1) aufgearbeitet wird, bevorzugt durch Waschen mit wässrigen Medien und anschließender Rektifikation,
und bei dem
die kontinuierliche Reaktion durch Beendigung der Zufuhr des Benzol-haltigen Stroms (a.1) und der Salpetersäure (a.3) unterbrochen wird und nach vollständiger Umsetzung der im Reaktor vorhandenen Restmengen an Salpetersäure zu Nitrobenzol und nach Entfernung der organischen Phase (b.2) aus dem Phasentrennapparat und nach Aufkonzentrierung von (b.1) zu (c.1) die verbleibende Schwefelsäure-haltige Phase (c.1) so lange bei einer Temperatur von 60 °C bis 140 °C durch den Reaktor, den Phasentrennapparat, den Verdampfungsapparat und den Schwefelsäuretank im Kreis gefahren wird, bis der Gehalt an organischen Verbindungen in (c.1) kleiner als 1,0 Massen-%, bezogen auf die Gesamtmasse der Schwefelsäure-haltigen Phase (c.1), ist, und die so gereinigte Schwefelsäure-haltige Phase (c.1) als Bestandteil von (a.2) des nächsten Produktionszyklus eingesetzt wird,
oder
ausgehend von einer nicht in Betrieb befindlichen Produktionsanlage vor der Zufuhr des Benzol-haltigen Stroms (a.1) und der Salpetersäure (a.3) die noch aus dem vorigen Produktionszyklus vorhandene Schwefelsäure-haltige Phase (c.1) so lange bei einer Temperatur von 60 °C bis 140 °C durch den Reaktor, den Phasentrennapparat, den Verdampfungsapparat und den Schwefelsäuretank im Kreis gefahren wird, bis der Gehalt an organischen Verbindungen in (c.1) kleiner als 1,0 Massen-%, bezogen auf die Gesamtmasse der Schwefelsäure-haltigen Phase (c.1), ist, und die so gereinigte Schwefelsäure-haltige Phase (c.1) als Bestandteil von (a.2) des neuen Produktionszyklus eingesetzt wird,
sodass
mindestens während des Zeitraums t dem Reaktor nur ein solcher Schwefelsäurestrom (a.2) zugeführt wird, der einen Gehalt an organischen Verbindungen von kleiner als 1,0 Massen-%,
bevorzugt von kleiner als 0,50 Massen-%, besonders bevorzugt von kleiner als 0,20 Massen-%, jeweils bezogen auf die Gesamtmasse von (a.2), aufweist.

Der *auf Salpetersäure bezogene Benzolüberschuss* von 2,0 % bis 20 %, bevorzugt von 5,0 % bis 10 % der Theorie, bezieht sich auf das molare Verhältnis von Benzol und Salpetersäure. Theoretisch reagiert ein Mol Salpetersäure mit einem Mol Benzol zu einem Mol Nitrobenzol.

Dem Fachmann ist bekannt, dass ein kontinuierlich betriebener industrieller Prozess ausgehend von einer nicht in Betrieb befindlichen Produktionsanlage (z. B. nach einem wartungsbedingten Stillstand) nicht augenblicklich wieder auf die Prozessparameter vor dem Produktionsstillstand hochgefahren werden kann. Edukte und Apparaturen müssen aufgeheizt werden, Apparate müssen ggf. inertisiert werden, die Belastung der Apparate mit den Edukten wird allmählich auf den angestrebten Soll-Wert gesteigert. Wenn eine Produktionsanlage zur Herstellung von Nitrobenzol bei einer *Soll-Belastung M'ₛₒₗₗ* von x [kg(Benzol)/h] betrieben werden soll, so kann diese Soll-Belastung beispielsweise erreicht werden, indem zu Beginn der Nitrierung die Belastung M' zunächst auf einen Wert von 0,25 x eingestellt und die Belastung dann über die Zwischenstufen M' = 0,50 x und M' = 0,75 x innerhalb von 4 Stunden auf den Wert M' = x = M'_{Soll} gesteigert wird. Alternativ kann auch von einem bestimmten Startwert aus, z. B. M' = 0,50 x, eine kontinuierliche Laststeigerung bis M' = x durchgeführt werden. Diese Beispiele stehen natürlich nur exemplarisch für eine Vielzahl möglicher Anfahrprozeduren, deren genaue Ausgestaltung von den konkreten Gegebenheiten einer Produktionsanlage abhängt und daher nicht verallgemeinert werden kann. Ein gemeinsames Merkmal aller denkbaren Anfahrprozeduren ist jedoch, dass erst nach Verstreichen eines Zeitraums t die angestrebte Soll-Belastung von x erreicht wird. Dieser Zeitraum t wird erfindungsgemäß als *Anfahrzeit* bezeichnet. Während der Anfahrzeit wird der dem Reaktor kontinuierlich zugeführte Mengenstrom an Salpetersäure (a.3) natürlich dem jeweiligen Mengenstrom an Benzol-haltigem Strom (a.1) angepasst, d. h., zu Beginn der Anfahrzeit, wenn dem Reaktor erst ein Bruchteil der angestrebten Soll-Belastung M'_{Soll} zugeführt wird, wird dem Reaktor auch nur ein entsprechender Bruchteil an Salpetersäure (a.3) zugeführt. Bevorzugt wird während der Anfahrzeit t der gleiche prozentuale Überschuss von Benzol bezogen auf Salpetersäure eingehalten wie nach Erreichen der Soll-Belastung M'_{Soll}. Das Massenverhältnis von Salpetersäure (a.3) zu Schwefelsäure (a.2) kann während der Anfahrzeit von demjenigen nach Erreichen der Soll-Belastung an Benzol M'_{Soll} abweichen; es kann insbesondere geringer sein. Insbesondere ist es bevorzugt, dem Reaktor zunächst nur Schwefelsäure (a.2) zuzuführen und erst nach Erreichen eines stabilen Betriebszustands des Schwefelsäurekreislaufs Salpetersäure (a.3) und Benzol-haltigen Strom (a.1) zuzuführen.

Erfindungsgemäß wird Salpetersäure unterstöchiometrisch eingesetzt, sodass der wässrige Anteil (b.1) des in Schritt a) erhaltenen rohen Reaktionsproduktes im Wesentlichen aus verdünnter Schwefelsäure besteht. Diese wird in Schritt c) aufkonzentriert, um eine Rezyklierung der Schwefelsäure zu ermöglichen. Im Allgemeinen kann die Schwefelsäure vollständig rezykliert werden, so dass auf eine Zugabe frischer Schwefelsäure in Schritt a) weitgehend bis vollständig verzichtet werden kann, d. h., bevorzugt entspricht der Schwefelsäurestrom (a.2) dem rezyklierten Schwefelsäurestrom (c.1), dem sog. *"Kreislaufschwefelsäurestrom"*, da er immer wieder verwendet und daher während der Reaktion im Kreis gefahren wird. Wenn es erforderlich ist, werden Schwefelsäureverluste durch entsprechende Zugabe frischer Schwefelsäure in Schritt a) ersetzt. Der Schwefelsäurestrom (a.2) setzt sich also aus Kreislaufschwefelsäure (c.1) und ggf. zugesetzter frischer Schwefelsäure zusammen. Bevorzugt enthält die Schwefelsäure (a.2) 95 Massen-% bis 100 Massen-% aufkonzentrierte Schwefelsäure (c.1), bezogen auf die Gesamtmasse von (a.2). Frische Schwefelsäure ist normalerweise frei von organischen Verbindungen, sodass das Augenmerk im Zusammenhang mit der vorliegenden Erfindung auf die Kreislaufschwefelsäure (c.1) zu richten ist. Diese immer im Kreis gefahrene Schwefelsäure reichert sich während des Betriebs mit organischen Verbindungen an. Nach dem Kenntnisstand aus dem Stand der Technik sollte dies kein Problem sein, da die Schwefelsäure ohnehin in Kontakt mit organischen Verbindungen, dem Edukt Benzol und nach beginnender Umsetzung dem Produkt Nitrobenzol, kommt. Wie weiter unten noch näher erläutert wird, wurde jedoch im Rahmen der vorliegenden Erfindung überraschend gefunden, dass eine Begrenzung des Organikagehaltes in der Kreislaufschwefelsäure positive Effekte hat.

*Organische Verbindungen* im Sinne der vorliegenden Erfindung sind bevorzugt ausgewählt aus der Gruppe bestehend aus Benzol, Nitrobenzol, den Isomeren des Dinitrobenzols und den Isomeren des Nitrophenols, wobei Nitrobenzol besonders bevorzugt ist. Erfindungsgemäß muss der Gehalt an organischen Verbindungen in der Kreislaufschwefelsäure kontrolliert werden. Zu diesem Zweck sind analytische Messungen erforderlich. Gemessen wird die Kreislaufschwefelsäure im Vorlagetank zur Reaktion, und zwar bevorzugt durch Probenahme an den entsprechenden Stellen und Analyse der Proben mittels Gaschromatographie. Andere Bestimmungsmethoden (z. B. spektroskopische Methoden), ggf. auch online oder inline, können, wenn auch nicht bevorzugt, grundsätzlich ebenfalls angewandt werden. Maßgeblich für die erfindungsgemäße Obergrenze des Gehaltes an organischen Verbindungen ist jedoch der durch Gaschromatographie ermittelte Wert.

Das Wort *"ein"* ist im Rahmen dieser Erfindung im Zusammenhang mit zählbaren Größen nur dann als Zahlwort zu verstehen, wenn dies ausdrücklich gesagt wird. Bspw. schließt der Ausdruck *"ein Reaktor"* die Möglichkeit des Vorhandenseins mehrerer (in Reihe oder parallel geschalteter) Reaktoren nicht aus.

Erfindungswesentlich ist nun, dass der Gehalt an organischen Verbindungen - speziell an Nitrobenzol - im Schwefelsäure-haltigen Strom (a.2) mindestens während der Anfahrzeit t den zuvor genannten Konzentrationen entspricht. Dieses Ziel kann auf alternative Weisen erreicht werden:

In einer **ersten Variante** wird nach Beendigung eines Produktionszyklus, d. h., nach Beendigung der Zufuhr von Benzol (a.1) und Salpetersäure (a.3) und vollständiger Umsetzung der noch in der Produktionsanlage befindlichen Restmengen zu Nitrobenzol eine Phasentrennung im Phasentrennapparat durchgeführt und die organische Phase, umfassend Benzol, Nitrobenzol und organischen Nebenprodukte, aus diesem entfernt. Somit minimiert sich die absolute Menge an Organika, die in dem Verdampfungsapparat zu entfernen sind, und es gelangen keine über die in der Kreislaufsäure gelösten Reste hinausgehenden Mengen an Organika über den Verdampfungsapparat in den Schwefelsäurevorlagetank. Dann wird die verbliebene aufkonzentrierte Schwefelsäure (c.1) so lange bei erhöhter Temperatur, bevorzugt bei einer Temperatur von 60 °C bis 140 °C, besonders bevorzugt bei 100°C bis 140°C, durch den Reaktor, den Phasentrennapparat, den Verdampfungsapparat und den Schwefelsäuretank im Kreis gefahren, wobei im Verdampfungsapparat nach Abstellen der Reaktion bevorzugt ein absoluter Druck von 50 mbar bis 300 mbar, besonders bevorzugt von 70 mbar bis 100 mbar und ganz besonders bevorzugt von 80 mbar bis 90 mbar, eingestellt wird, bis der geforderte Höchstgehalt an organischen Verbindungen im Kreislaufschwefelsäurestrom (c.1) von kleiner als 1,0 Massen-%, bevorzugt von kleiner als 0,50 Massen-%, besonders bevorzugt von kleiner als 0,20 Massen-%, jeweils bezogen auf die Gesamtmasse der Kreislaufschwefelsäure (c.1), erreicht wird. Die organischen Verbindungen gelangen dabei in die Gasphase und werden aus dem Verdampfungsapparat abgeführt. Um das Austreiben der organischen Verbindungen, insbesondere von Nitrobenzol, aus der Kreislaufschwefelsäure (c.1) zu unterstützen, können dieser Schwefelsäure geringe Mengen an Wasser (bevorzugt 0,1 Massen-% bis 2 Massen-%, bezogen auf die Gesamtmasse der Kreislaufschwefelsäure (c.1)) zugegeben werden. Als Wasser wird dabei bevorzugt vollentsalztes Wasser (VE-Wasser), besonders bevorzugt eine Mischung aus VE-Wasser und Dampfkondensat (d. i. ein Kondensat von Wasserdampf, welcher durch Wärmeaustausch von Wasser mit beliebigen exothermen Verfahrensschritten erhalten wurde) und ganz besonders bevorzugt Dampfkondensat eingesetzt. Beim erneuten Anfahren der Reaktion wird dann die im Schwefelsäuretank befindliche Schwefelsäure (c.1), ggf. nach Zugabe von frischer Schwefelsäure, als Strom (a.2) in den Reaktor geführt. Da frische Schwefelsäure organische Verbindungen entweder gar nicht oder nur in unbedeutenden Spuren enthält, wird so gewährleistet, das hinreichend lange, d. h., mindestens während der Anfahrzeit, die erfindungsgemäßen Anforderungen an den Maximalgehalt an organischen Verbindungen in Strom (a.2) eingehalten werden. Gegenstand der Erfindung ist daher insbesondere auch ein Verfahren, bei dem die kontinuierliche Reaktion durch Beendigung der Zufuhr des Benzol-haltigen Stroms (a.1) und der Salpetersäure (a.3) unterbrochen und nach vollständiger Umsetzung der im Reaktor vorhandenen Restmengen an Salpetersäure zu Nitrobenzol und nach Entfernung der organischen Phase (b.2) aus dem Phasentrennapparat und nach Aufkonzentrierung von (b.1) zu (c.1) die verbleibende Schwefelsäure-haltige Phase (c.1) so lange bei einer Temperatur von 60 °C bis 140 °C, bevorzugt von 100 °C bis 140 °C, durch den Reaktor, den Phasentrennapparat, den Verdampfungsapparat und den Schwefelsäuretank im Kreis gefahren wird, wobei bevorzugt im Verdampfungsapparat ein absoluter Druck von 50 mbar bis 300 mbar, besonders bevorzugt von 70 mbar bis 100 mbar, ganz besonders bevorzugt von 80 mbar bis 90 mbar eingestellt wird, bis der Gehalt an organischen Verbindungen in (c.1) kleiner als 1,0 Massen-%, bevorzugt kleiner als 0,50 Massen-%, besonders bevorzugt kleiner als 0,20 Massen-%, jeweils bezogen auf die Gesamtmasse der Schwefelsäure-haltigen Phase (c.1), ist, und bei dem die so gereinigte Schwefelsäure-haltige Phase (c.1) als Bestandteil von (a.2) des nächsten Produktionszyklus eingesetzt wird.

In einer **zweiten Variante** wird vor Beginn eines neuen Produktionszyklus, d. h., vor der Zufuhr von Benzol (a.1) und Salpetersäure (a.3), die noch aus dem letzten Produktionszyklus verbliebene aufkonzentrierte Schwefelsäure (c.1) so lange bei erhöhter Temperatur, bevorzugt bei einer Temperatur von 60 °C bis 140 °C, besonders bevorzugt bei einer Temperatur von 80 °C bis 120 °C und ganz besonders bevorzugt 90 °C bis 110 °C, durch den Reaktor, den Phasentrennapparat, den Verdampfungsapparat und den Schwefelsäuretank im Kreis gefahren wird, wobei im Verdampfungsapparat bevorzugt ein absoluter Druck von 50 mbar bis 300 mbar, besonders bevorzugt von 70 mbar bis 100 mbar, ganz besonders bevorzugt 80 mbar bis 90 mbar, eingestellt wird, bis der geforderte Höchstgehalt an organischen Verbindungen im Kreislaufschwefelsäurestrom (c.1) von kleiner als 1,0 Massen-%, bevorzugt von kleiner als 0,50 Massen-%, besonders bevorzugt von kleiner als 0,20 Massen-%, jeweils bezogen auf die Gesamtmasse der Kreislaufschwefelsäure (c.1), erreicht wird. Die organischen Verbindungen gelangen dabei in die Gasphase und werden aus dem Verdampfungsapparat abgeführt. Beim erneuten Anfahren der Reaktion wird dann die im Schwefelsäuretank befindliche Schwefelsäure (c.1), ggf. nach Zugabe von frischer Schwefelsäure, als Strom (a.2) in den Reaktor geführt. Da frische Schwefelsäure organische Verbindungen entweder gar nicht oder nur in unbedeutenden Spuren enthält, wird so gewährleistet, das hinreichend lange, d. h., mindestens während der Anfahrzeit, die erfindungsgemäßen Anforderungen an den Maximalgehalt an organischen Verbindungen in Strom (a.2) eingehalten werden. Gegenstand der Erfindung ist daher insbesondere auch ein Verfahren, bei dem ausgehend von einer nicht in Betrieb befindlichen Produktionsanlage vor Beginn eines neuen Produktionszyklus, d. h., vor Beginn der Zufuhr des Benzol-haltigen Stroms (a.1) und der Salpetersäure (a.3), die noch aus dem vorigen Produktionszyklus vorhandene Schwefelsäure-haltige Phase (c.1) so lange bei einer Temperatur von 60 °C bis 140°C, bevorzugt 80 °C bis 120 °C, besonders bevorzugt 90 °C bis 110 °C durch den Reaktor, den Phasentrennapparat, den Verdampfungsapparat und den Schwefelsäuretank im Kreis gefahren wird, bis der Gehalt an organischen Verbindungen in (c.1) kleiner als 1,0 Massen-%, bevorzugt kleiner als 0,50 Massen-%, besonders bevorzugt kleiner als 0,20 Massen-%, jeweils bezogen auf die Gesamtmasse der Schwefelsäure-haltigen Phase (c.1), ist, und bei dem die so gereinigte Schwefelsäure-haltige Phase (c.1) als Bestandteil von (a.2) des neuen Produktionszyklus eingesetzt wird.

Beide Varianten unterscheiden sich demnach im Zeitpunkt, zu dem die Kreislaufschwefelsäure bei erhöhter Temperatur im Kreis gefahren ("ausgekocht") wird. Die zweite Variante kommt bevorzugt dann zum Einsatz, wenn es unplanmäßig zu einer Abstellung der Nitrieranlage gekommen ist. Beide Varianten können auch kombiniert werden.

Nachstehend werden noch die Schritte a) bis d) der Erfindung näher erläutert. Verschiedene Ausführungsformen sind dabei beliebig miteinander kombinierbar, solange sich für den Fachmann aus dem Kontext nicht eindeutig das Gegenteil ergibt.

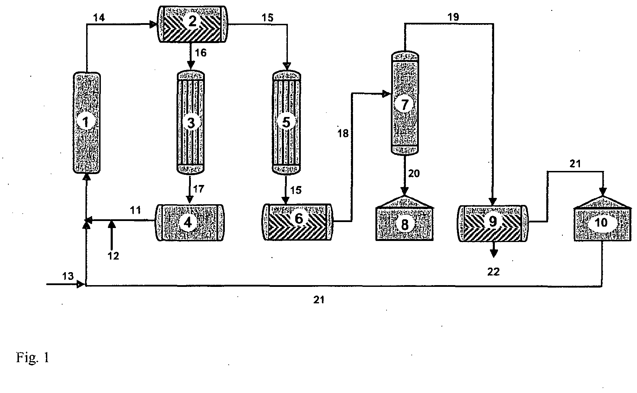

Schritt a) kann grundsätzlich nach allen aus dem Stand der Technik bekannten adiabaten Nitrierverfahren durchgeführt werden, solange mit diesen die spezifizierten Randbedingungen bzgl. des Benzolüberschusses und der Reinheit der Einsatzstoffe und Hilfsstoffe eingehalten werden können. Für die Ausführung dieses Schrittes des erfindungsgemäßen Verfahrens wird bevorzugt ein Rohrreaktor eingesetzt, in dem mehrere Dispergierelemente über die Länge des Reaktors verteilt angeordnet sind, die eine intensive Dispergierung und Durchmischung von Benzol, Salpetersäure und Schwefelsäure gewährleisten. Ein solcher Reaktor, sowie die Form einsetzbarer Dispergierelemente, sind beispielsweise in EP 0708 076 B1 (Figur 2) und EP 1 291 078 A2 (Figur 1) beschrieben. Bevorzugt wird Schritt a) in einer Verfahrensführung ausgeführt, wie sie in DE 10 2008 048 713 A1, insbesondere Absatz [0024], beschrieben ist.

Die Phasentrennung in **Schritt b)** erfolgt ebenfalls nach aus dem Stand der Technik an sich bekannten Verfahren in einem dem Fachmann bekannten Trennbehälter. Die wässrige Phase (b.1) enthält im Wesentlichen (infolge der Bildung von Reaktionswasser und durch das Einschleppen von Wasser in die Reaktion aus der eingesetzten Salpetersäure) verdünnte Schwefelsäure neben anorganischen Verunreinigungen, die organische Phase (b.2) enthält im Wesentlichen Nitrobenzol neben überschüssigem Benzol und organischen Verunreinigungen.

Die Aufkonzentrierung der wässrigen Phase (b.1) in **Schritt c)** erfolgt grundsätzlich wie aus dem Stand der Technik bekannt. Die Schwefelsäure in der wässrigen Phase wird in einem Entspannungsverdampfer (auch als Blitzverdampfer bezeichnet) aufkonzentriert, indem Wasser in einen Bereich reduzierten Druckes hinein verdampft wird. Bei richtiger Wahl der Reaktionsbedingungen in der adiabaten Nitrierung von Benzol mit Mischsäure erzielt man mit der Reaktionswärme der exothermen Reaktion eine so starke Erhitzung der Schwefelsäure enthaltenden wässrigen Phase (b.1), dass im Entspannungsverdampfer gleichzeitig wieder die Konzentration und Temperatur der Schwefelsäure enthaltenden wässrigen Phase eingestellt werden kann, die diese vor der Reaktion mit Benzol und Salpetersäure bei Eintritt in den Reaktorraum hatte, d. h., (c.1) entspricht hinsichtlich Temperatur und Konzentration (a.2). Dies ist beschrieben in EP 2 354 117 A1, insbesondere Absatz [0045].

Die Aufarbeitung der organischen Phase (b.2) in **Schritt d)** erfolgt grundsätzlich wie aus dem Stand der Technik bekannt. Eine bevorzugte Verfahrensweise wird nachstehend geschildert:

Die organische Phase (b.2), die üblicherweise noch Spuren an Säure enthält, wird in einer bis zwei Wäschen, bevorzugt einer Wäsche, mit einer wässrigen Waschflüssigkeit gewaschen und dann von der sauren wässrigen Phase durch Phasentrennung getrennt (bei mehreren Wäschen nach jeder einzelnen Wäsche). Bei diesem Vorgang werden die Säurereste, die das rohe Nitrobenzol (b.2) enthält, ausgewaschen; daher wird dieser Verfahrensschritt auch als *saure Wäsche* bezeichnet. Dieser Schritt ist aus dem Stand der Technik hinlänglich bekannt und wird daher hier nur kurz umrissen. Bevorzugt werden zur Durchführung dieser sauren Wäsche im Betrieb anfallende wässrige Ströme rezyklisiert. (**Schritt d(i)**.)

Die so erhaltene organische Phase wird anschließend in einer bis zwei, bevorzugt einer alkalischen Wäsche(n) mit einer wässrigen Lösung einer Base, bevorzugt ausgewählt aus der Gruppe bestehend aus Natriumhydroxid, Natriumcarbonat und Natriumhydrogencarbonat gewaschen und anschließend von dem alkalischen Waschwasser durch Phasentrennung getrennt (bei mehreren Wäschen nach jeder einzelnen Wäsche). Besonders bevorzugt wird als wässrige Baselösung Natronlauge eingesetzt. Dieser Schritt ist aus dem Stand der Technik hinlänglich bekannt und wird daher hier nur kurz umrissen. Der pH-Wert der eingesetzten Natronlauge und ihr Massenverhältnis zur organischen Phase werden so eingestellt, dass acide Verunreinigungen (z. B. als Nebenprodukte gebildete Nitrophenole und in Schritt b) nicht vollständig entfernte Säurereste) in Schritt c) weitgehend bis vollständig, bevorzugt vollständig, neutralisiert werden. Die anschließende Aufarbeitung des alkalischen Abwassers kann nach den Verfahren des Standes der Technik, z. B. gemäß EP 1 593 654 A1 und EP 1 132 347 A2, erfolgen. (**Schritt d(ii)**.)

Die so erhaltene organische Phase wird schließlich in mindestens einer, bevorzugt zwei bis vier, besonders bevorzugt zwei bis drei, ganz besonders bevorzugt zwei, neutralen Wäsche(n) mit Wasser gewaschen und anschließend von der wässrigen Phase durch Phasentrennung getrennt (bei mehreren Wäschen nach jeder einzelnen Wäsche). Dies kann grundsätzlich nach allen im Stand der Technik üblichen Verfahren geschehen. Als Waschwasser wird dabei bevorzugt vollentsalztes Wasser (VE-Wasser), besonders bevorzugt eine Mischung aus VE-Wasser und Dampfkondensat (d. i. ein Kondensat von Wasserdampf, welcher durch Wärmeaustausch von Wasser mit beliebigen exothermen Verfahrensschritten erhalten wurde) und ganz besonders bevorzugt Dampfkondensat eingesetzt. Bevorzugt ist eine Verfahrensweise, bei der in der letzten neutralen Wäsche eine Elektrophorese eingesetzt wird (siehe WO 2012/013678 A2). (**Schritt d(iii)**.)

Das gewaschene Nitrobenzol wird schließlich von gelöstem Wasser, nicht umgesetztem Benzol und ggf. organischen Verunreinigungen durch weitere Aufarbeitung befreit. Diese Aufarbeitung erfolgt bevorzugt durch Destillation, wobei über Kopf die Brüden Wasser und Benzol und ggf. organische Verunreinigungen ausgetrieben werden. Die Brüden werden gekühlt und in einen Trennbehälter gefahren. In der unteren Phase setzt sich Wasser ab, das abgetrennt wird. In der oberen Phase befinden sich Benzol und Leichtsieder, die als Rückbenzol (d.2) wieder der Reaktion zugeführt werden. Bevorzugt wird als Destillationsapparat eine Rektifizierkolonne eingesetzt. Das Sumpfprodukt der Destillation wird, ggf. nach einer weiteren Destillation, in welcher Nitrobenzol *als Destillat* (also als Kopf- oder Seitenstromprodukt) erhalten wird, als Rein-Nitrobenzol (d.1) weiteren Anwendungen (wie der Hydrierung zu Anilin) zugeführt. (Schritt d(iv).)

Durch die erfindungsgemäßen Vorgehensweisen ergeben sich die folgenden Vorteile für das Anfahrprocedere der adiabaten Nitrierung:
i) Die Reaktionsmischung heizt sich schneller auf, weil nicht noch zusätzlich die organischen Verunreinigungen in der Kreislaufschwefelsäure aufgeheizt werden müssen. Dadurch kann früher auf den reaktionsunterstützenden Einsatz von Dampf verzichtet werden.
ii) Die Benzolumsätze sind optimal und nur die gegenüber der theoretischen Menge überschüssige Menge an Benzol, nicht jedoch weiteres, durch unvollständige Reaktion vorhandenes Benzol belastet die Aufarbeitung in Schritt d).
iii) Die Bildung von Nebenprodukten in der Reaktion wie Dinitrobenzol und Pikrinsäure wird minimiert, weil zwischen Salpetersäuredosierung und Benzoldosierung am Reaktoreingang kein Nitrobenzol in signifikanter Menge vorhanden ist, das mit dem dort vorhandenen großen Überschuss an Salpetersäure Nebenreaktionen eingehen könnte.
iv) Die Abwesenheit von organischen Verunreinigungen in der Kreislaufschwefelsäure beim Anfahren der Nitrierung von Benzol hat außerdem den Vorteil, dass die hydraulische Belastung in der Reaktion geringer ausfällt und somit die Reaktion auch schneller auf Soll-Last hochgefahren werden kann.

Somit ermöglicht das erfindungsgemäße Verfahren durch Verwendung von Schwefelsäure (a.2) mit einem Gehalt an organischen Verbindungen von kleiner als 1,0 Massen-%, bevorzugt von kleiner als 0,50 Massen-%, besonders bevorzugt von kleiner als 0,20 Massen-%, jeweils bezogen auf die Gesamtmasse von (a.2), das Anfahren der adiabaten Nitrierung von Benzol und die anschließende Aufarbeitung des entstandenen Rohnitrobenzols in technisch reibungsloser Weise ohne Ausfallzeiten mit direkt hoher Endproduktqualität. Nach Verstreichen der Anfahrzeit ist das Einhalten dieser Obergrenzen für den Gehalt an organischen Verbindungen nicht mehr zwingend erforderlich, aber dennoch von Vorteil.

### Beispiele

Gehaltsangaben in ppm oder % sind Massenanteile bezogen auf die Gesamtmasse des jeweiligen Stoff(strom)s. Analysenwerte wurden, sofern nicht anders angegeben, mittels Gaschromatographie bestimmt.

### Allgemeine Bedingungen für die Herstellung von Nitrobenzol bei eingefahrener Produktionsanlage (nach Verstreichen der Anfahrzeit t)

### (Siehe Figur 1)

Einem Reaktor (1) werden ein Schwefelsäure- (11), ein Salpetersäure- (12), ein Frischbenzol- (13) und ein Rückbenzolstrom (21) zugeführt. Es wird ein 5 bis 10%iger Überschuss an Benzol, bezogen auf Salpetersäure, eingesetzt. Von diesem Überschuss und der Qualität des Einsatzbenzols hängt die anfallende Menge an Rückbenzol ab. Nach vollständiger Umsetzung der Salpetersäure mit dem Benzol zu Nitrobenzol unter adiabater Reaktionsführung wird das nunmehr ca. 130°C heiße Reaktionsprodukt (14) einem Phasentrennapparat (2) zugeführt, in dem das Reaktionsprodukt (14) in eine organische Phase ((15),=Roh-Nitrobenzol, enthaltend neben Nitrobenzol noch Benzol und Leichtsieder) und eine wässrige Phase ((16), = Absäure, enthaltend neben Schwefelsäure noch geringe Anteile an Nitrobenzol und Benzol) zerfällt. Die wässrige, hauptsächlich Schwefelsäure umfassende Phase (16) wird im Verdampfer (3) durch plötzliche Druckerniedrigung einer Blitzverdampfung von Wasser unterzogen und auf diese Weise aufkonzentriert. Die aufkonzentrierte Schwefelsäure (17) wird im Schwefelsäuretank (4) zur erneuten Verwendung gelagert. Nach dem Abtrennen im Phasentrennapparat wird das Rohnitrobenzol (15) in der Rohnitrobenzolkühlung (5) auf ca. 50 °C abgekühlt und der Wäsche (6) zugeführt. Der so erhaltene von Nitrophenolen und Salzen weitgehend befreite Strom gereinigten Roh-Nitrobenzols (18) wird wieder aufgeheizt und in einer Destillationskolonne (7) von Wasser, Benzol und anderen Leichtsiedern, welche über Kopf abgetrennt werden (19), befreit, wodurch getrocknetes Rein-Nitrobenzol (20) erhalten und in Tank (8) gelagert wird. Das kondensierte Kopfprodukt (19) der Destillationskolonne (7) wird einem Phasentrennapparat (9) zugeführt, in dem das Kopfprodukt in eine organische Phase ((21), enthaltend Benzol und Leichtsieder) und eine wässrige Phase (22) zerfällt. Die organische Phase (21) wird in einem Puffertank (10) zwischengelagert und von dort, wie oben schon beschrieben, zur Reaktion in den Zulauf des Reaktors (1) zurück gefahren.

So hergestelltes Nitrobenzol hat typischerweise eine Reinheit von ca. 99,96 % (GC), einen Restbenzolgehalt von 0,0028 % (GC), einen Gehalt an 1,3-Dinitrobenzol von 0,0273 % (GC) und einen Nitroplaenolgehalt von < 5 ppm (HPLC). Ferner weist das Nitrobenzol einen Wassergehalt von 0,0079 % (Karl-Fischer) auf.

### Allgemeine Bedingungen für das Anfahren eines adiabaten Nitrobenzol-Prozesses

### (Siehe Figur 1)

Die Kreislaufschwefelsäurepumpe wird gestartet, und Schwefelsäure aus dem Schwefelsäuretank (4) wird in den Reaktor (1) gefahren, läuft anschließend über in den Phasentrennapparat (2) und von dort in den Verdampfungsapparat (3), um abschließend wieder im Schwefelsäuretank (4) anzukommen. Der Druck im Verdampfungsapparat wird reduziert. Um schonend mit den in der Nitrierreaktion eingesetzten metallischen Schwefelsäurekreislaufpumpen umzugehen, wird beim Warmfahren der Anlage mit Schwefelsäure immer auch in Spuren Salpetersäure mitgefahren, um die Schwefelsäurekreislaufpumpe zu passivieren und die Zerstörung der Pumpe durch Korrosion zu verhindern. Dies ist nicht notwendig, wenn Kunststoffpumpen verwendet werden. In kontinuierlicher Fahrweise wird die Schwefelsäure mit indirektem Dampf auf eine Temperatur von 101 °C aufgeheizt. Während des Aufheizens wird die Schwefelsäure durch den Schwefelsäuretank, den Reaktor, den Phasentrennapparat und den Verdampfungsapparat im Kreis gefahren, wobei der absolute Druck im Verdampfungsapparat 85 mbar beträgt. Sobald die Soll-Temperatur der Kreislaufschwefelsäure erreicht ist und (Beispiele 2 bis 4) ein erfindungsgemäßer Gehalt an organischen Verbindungen in der Kreislaufschwefelsäure vorliegt, wird die Nitrierung angefahren. Im Vergleichsbeispiel 1 wurde die Bedingung im Hinblick auf den Gehalt an organischen Verbindungen in der Kreislaufschwefelsäure nicht eingehalten (Gehalt an organischen Verbindungen größer als 1,0 Massen-%). Zum Anfahren der Nitrierung wird ein Benzolstrom (aus 13 (Frischbenzol) und gegebenenfalls 21 (Rückbenzol)) sowie der Salpetersäurestrom (12) zeitgleich zum Reaktoreingang gefahren, wo die Nitrierung mit der Dispergierung der Einsatzstoffe beginnt. Um die Nennkapazität der Anlage zu erreichen (M'_{Soll}), wird zunächst mit geringeren Mengenströmen an Benzol und Salpetersäure gestartet (in den Beispielen 1 bis 4 wurde die Anlage mit 50 % der Nennkapazität gestartet, was einer Produktionsleistung von 30 t/h (Nitrobenzol) entsprach). Diese Mengenströme werden dann während einer Anfahrzeit t auf die Nennlast erhöht. Das Hochfahren der Anlage kann manuell oder mit einer Anfahrautomatik gestaltet werden. Die Anlage wurde jeweils so schnell wie möglich auf die Nennlast hochgefahren, wobei darauf geachtet wurde, einen vollständigen Umsatz der Salpetersäure zu erzielen.

**Tabelle 1: Gegenüberstellung der Ergebnisse aus den Beispielen**

| Beispiel | Nitrobenzolgehalt in Strom 11^{[a]} | Dinitrobenzolgehalt in Strom 15^{[a]} | Pikrinsäuregehalt in Strom 15^{[a]} |
|---|---|---|---|
| 1 (Vergleich) | 1,3 Massen-% | 310 ppm | 321 ppm |
| 2 (erfindungsgemäß) | 0,95 Massen-% | 251 ppm | 240 ppm |
| 3 (erfindungsgemäß) | 0,45 Massen-% | 195 ppm | 132 ppm |
| 4 (erfindungsgemäß) | 0,21 Massen-% | 187 ppm | 127 ppm |
| [a] Durchschnittswerte über die Anfahrzeit. | | | |

Wie die Beispiele zeigen, entstehen bei erhöhtem Nitrobenzolgehalt in der Kreislaufschwefelsäure auch große Mengen an Nebenprodukten (Beispiel 1). Bei erfindungsgemäßer Vorgehensweise wird demgegenüber die Entstehung von Nebenprodukten signifikant reduziert, und ein späteres Verschneiden der Anfahrware mit reineren Nitrobenzolchargen kann unterbleiben.

## Patentansprüche

1. Kontinuierliches Verfahren zur Herstellung von Nitrobenzol durch Nitrierung von Benzol, bei dem
a) ein Benzol-haltiger Strom (a.1) in einem Reaktor mit einem Gemisch aus Schwefelsäure (a.2) und Salpetersäure (a.3) unter adiabaten Bedingungen umgesetzt wird, wobei Benzol in einem, stöchiometrischen Überschuss bezogen auf Salpetersäure (a.3) eingesetzt wird, und wobei die Menge M' des dem Reaktor pro Stunde zugeführten Benzol-haltigen Stroms (a.1) innerhalb eines Zeitraums t von Beginn der Nitrierung bis zum Erreichen eines vorgegebenen Soll-Werts für M' gesteigert wird,
b) das in Schritt a) erhaltene Verfahrensprodukt in einem Phasentrennapparat in eine wässrige, Schwefelsäure umfassende Phase (b.1) und eine organische, Nitrobenzol umfassende Phase (b.2) getrennt wird,
c) die in Schritt b) erhaltene wässrige Phase (b.1) durch Verdampfung von Wasser in einem Verdampfungsapparat zu einer wässrigen Schwefelsäure-haltigen Phase (c.1) mit gegenüber (b.1) erhöhter Schwefelsäurekonzentration aufkonzentriert wird, und wobei die Phase (c.1) über einen Schwefelsäuretank in Schritt a) zurückgeführt und als Bestandteil von (a.2) eingesetzt wird,
d) die in Schritt b) erhaltene organische Phase (b.2) zu Rein-Nitrobenzol (d.1) aufgearbeitet wird,
**dadurch gekennzeichnet, dass**
die kontinuierliche Reaktion durch Beendigung der Zufuhr des Benzol-haltigen Stroms (a.1) und der Salpetersäure (a.3) unterbrochen wird und nach vollständiger Umsetzung der im Reaktor vorhandenen Restmengen an Salpetersäure zu Nitrobenzol und nach Entfernung der organischen Phase (b.2) aus dem Phasentrennapparat und nach Aufkonzentrierung von (b.1) zu (c.1) die verbleibende Schwefelsäure-haltige Phase (c.1) so lange bei einer Temperatur von 60 °C bis 140 °C durch den Reaktor, den Phasentrennapparat, den Verdampfungsapparat und den Schwefelsäuretank im Kreis gefahren wird, bis der Gehalt an organischen Verbindungen in (c.1) kleiner als 1,0 Massen-%, bezogen auf die Gesamtmasse der Schwefelsäure-haltigen Phase (c.1), ist, und die so gereinigte Schwefelsäure-haltige Phase (c.1) als Bestandteil von (a.2) des nächsten Produktionszyklus eingesetzt wird,
oder
ausgehend von einer nicht in Betrieb befindlichen Produktionsanlage vor der Zufuhr des Benzol-haltigen Stroms (a.1) und der Salpetersäure (a.3) die noch aus dem vorigen Produktionszyklus vorhandene Schwefelsäure-haltige Phase (c.1) so lange bei einer Temperatur von 60 °C bis 140 °C durch den Reaktor, den Phasentrennapparat, den Verdampfungsapparat und den Schwefelsäuretank im Kreis gefahren wird, bis der Gehalt an organischen Verbindungen in (c.1) kleiner als 1,0 Massen-%, bezogen auf die Gesamtmasse der Schwefelsäure-haltigen Phase (c.1), ist, und die so gereinigte Schwefelsäure-haltige Phase (c.1) als Bestandteil von (a.2) des neuen Produktionszyklus eingesetzt wird,
sodass
mindestens während des Zeitraums t dem Reaktor nur ein solcher Schwefelsäurestromstrom (a.2) zugeführt wird, der einen Gehalt an organischen Verbindungen von kleiner als 1,0 Massen-%, bezogen auf die Gesamtmasse von (a.2), aufweist.

2. Verfahren nach Anspruch 1, bei dem die organischen Verbindungen ausgewählt sind aus der Gruppe bestehend aus Benzol, Nitrobenzol, den Isomeren des Dinitrobenzols und den Isomeren des Nitrophenols.

3. Verfahren nach Anspruch 2, bei dem die organische Verbindung Nitrobenzol ist.

4. Verfahren nach einem der Ansprüche 1 bis 3, bei dem im Verdampfungsapparat ein absoluter Druck von 50 mbar bis 300 mbar eingestellt wird, während die Schwefelsäure-haltige Phase (c.1) im Kreis gefahren wird, um den Gehalt an organischen Verbindungen in (c.1) auf einen Wert von kleiner als 1,0 Massen-%, bezogen auf die Gesamtmasse der Schwefelsäure-haltigen Phase (c.1), zu bringen.

5. Verfahren nach einem der Ansprüche 1 bis 4, bei dem Benzol in Schritt a) in einem Überschuss von 2,0 % bis 20 % der Theorie eingesetzt wird.

## Claims

1. Continuous process for the preparation of nitrobenzene by the nitration of benzene, wherein
a) a benzene-containing stream (a.1) is reacted in a reactor with a mixture of sulfuric acid (a.2) and nitric acid (a.3) under adiabatic conditions, benzene being used in a stoichiometric excess, based on nitric acid (a.3), and the quantity M' of the benzene-containing stream (a.1) fed into the reactor per hour being increased over a period of time t from the beginning of the nitration until a preset target value for M' is achieved,
b) the process product obtained in step a) is separated in a phase separation apparatus into an aqueous phase (b.1) comprising sulfuric acid and an organic phase (b.2) comprising nitrobenzene,
c) the aqueous phase (b.1) obtained in step b) is concentrated by evaporation of water in an evaporation apparatus to give an aqueous phase (c.1) comprising sulfuric acid and having a higher sulfuric acid concentration than (b.1), and the phase (c.1) being recycled into step a) via a sulfuric acid tank and used as a component of (a.2),
d) the organic phase (b.2) obtained in step b) is worked up to pure nitrobenzene (d.1),
**characterized in that**
the continuous reaction is interrupted by stopping the addition of the benzene-containing stream (a.1) and the nitric acid (a.3) and, after complete conversion to nitrobenzene of the residual amounts of nitric acid present in the reactor and after removal of the organic phase (b.2) from the phase separation apparatus and after concentrating (b.1) to (c.1), the remaining sulfuric acid-containing phase (c.1) is circulated through the reactor, the phase separation apparatus, the evaporation apparatus and the sulfuric acid tank at a temperature of 60°C to 140°C until the content of organic compounds in (c.1) is lower than 1.0 mass%, based on the total mass of the sulfuric acid-containing phase (c.1), and the so purified sulfuric acid-containing phase (c.1) is used as a component of (a.2) in the next production cycle,
or
starting from a production plant that is not in operation, before the introduction of the benzene-containing stream (a.1) and the nitric acid (a.3), the sulfuric acid-containing phase (c.1) which is still there from the previous production cycle is circulated through the reactor, the phase separation apparatus, the evaporation apparatus and the sulfuric acid tank at a temperature of 60°C to 140°C until the content of organic compounds in (c.1) is lower than 1.0 mass%, based on the total mass of the sulfuric acid-containing phase (c.1), and the so purified sulfuric acid-containing phase (c.1) is used as a component of (a.2) in the new production cycle,
such that
at least during the period of time t, only such a sulfuric acid stream (a.2) having a content of organic compounds of less than 1.0 mass%, based on the total mass of (a.2), is fed into the reactor.

2. Process according to Claim 1, wherein the organic compounds are selected from the group consisting of benzene, nitrobenzene, the isomers of dinitrobenzene and the isomers of nitrophenol.

3. Process according to Claim 2, wherein the organic compound is nitrobenzene.

4. Process according to one of Claims 1 to 3, wherein the absolute pressure in the evaporation apparatus is adjusted to from 50 mbar to 300 mbar, while the sulfuric acid-containing phase (c.1) is circulated, in order to bring the content of organic compounds in (c.1) to a value of less than 1.0 mass%, based on the total mass of the sulfuric acid-containing phase (c.1).

5. Process according to one of Claims 1 to 4, wherein benzene is used in step a) in an excess of 2.0% to 20% of theory.

## Revendications

1. Procédé continu pour la préparation de nitrobenzène par nitruration de benzène, dans lequel
a) on transforme un flux (a.1) contenant du benzène dans un réacteur avec un mélange d'acide sulfurique (a.2) et d'acide nitrique (a.3) dans des conditions adiabatiques, le benzène étant utilisé en un excès stœchiométrique par rapport à l'acide nitrique (a.3) et la quantité M' du flux (a.1) contenant du benzène introduit dans le réacteur par heure étant augmentée dans une période t depuis le début de la nitruration jusqu'atteindre une valeur de consigne déterminée pour M',
b) on sépare le produit de procédé obtenu dans l'étape a) dans un appareil de séparation de phases en une phase (b.1) aqueuse, contenant de l'acide sulfurique et une phase (b.2) organique, contenant du nitrobenzène,
c) on concentre la phase (b.1) aqueuse obtenue dans l'étape b) par évaporation de l'eau dans un appareil d'évaporation en une phase (c.1) aqueuse contenant de l'acide sulfurique présentant une concentration en acide sulfurique augmentée par rapport à (b.1), la phase (c.1) étant recyclée via un réservoir d'acide sulfurique dans l'étape a) et utilisée comme constituant de (a.2),
d) on transforme la phase (b.2) organique obtenue dans l'étape b) en nitrobenzène (d.1) pur,
**caractérisé en ce que**
- la réaction continue est interrompue par l'arrêt de l'alimentation en flux (a.1) contenant du benzène et en acide nitrique (a.3) et, après transformation complète des quantités résiduelles présentes dans le réacteur d'acide nitrique en nitrobenzène et après élimination de la phase (b.2) organique de l'appareil de séparation de phases et après concentration de (b.1) en (c.1), la phase (c.1) contenant de l'acide sulfurique qui reste est mise en circulation à travers le réacteur, l'appareil de séparation de phases, l'appareil d'évaporation et le réservoir d'acide sulfurique à une température de 60°C à 140°C jusqu'à ce que la teneur en composés organiques dans (c.1) soit inférieure à 1,0% en masse, par rapport à la masse totale de la phase (c.1) contenant de l'acide sulfurique, et la phase (c.1) contenant de l'acide sulfurique ainsi purifiée est utilisée comme constituant de (a.2) du cycle de production suivant, ou
- partant d'une installation de production qui n'est pas en fonctionnement avant l'introduction du flux (a.1) contenant du benzène et de l'acide nitrique (a.3), la phase (c.1) contenant de l'acide sulfurique encore présente du cycle de production précédant est mise en circulation à travers le réacteur, l'appareil de séparation de phases, l'appareil d'évaporation et le réservoir d'acide sulfurique à une température de 60°C à 140°C jusqu'à ce que la teneur en composés organiques dans (c.1) soit inférieure à 1,0% en masse, par rapport à la masse totale de la phase (c.1) contenant de l'acide sulfurique, et la phase (c.1) contenant de l'acide sulfurique ainsi purifiée est utilisée comme constituant de (a.2) du nouveau cycle de production, de telle sorte que
- au moins pendant la période t, on alimente le réacteur uniquement en un flux (a.2) d'acide sulfurique qui présente une teneur en composés organiques inférieure à 1,0% en masse, par rapport à la masse totale de (a.2).

2. Procédé selon la revendication 1, dans lequel les composés organiques sont choisis dans le groupe constitué par le benzène, le nitrobenzène, les isomères du dinitrobenzène et les isomères du nitrophénol.

3. Procédé selon la revendication 2, dans lequel le composé organique est le nitrobenzène.

4. Procédé selon l'une quelconque des revendications 1 à 3, dans lequel on règle une pression absolue de 50 mbars à 300 mbars dans l'appareil d'évaporation pendant que la phase (c.1) contenant de l'acide sulfurique est mise en circulation pour amener la teneur en composés organiques dans (c.1) à une valeur inférieure à 1,0% en masse, par rapport à la masse totale de la phase (c.1) contenant de l'acide sulfurique.

5. Procédé selon l'une quelconque des revendications 1 à 4, dans lequel le benzène est utilisé dans l'étape a) en un excès de 2,0% à 20% de la théorie.
